(19) Europäisches Patentamt
European Patent Office
Office européen des brevets

(11) **EP 2 857 383 A1**

(12) **EUROPÄISCHE PATENTANMELDUNG**

(43) Veröffentlichungstag:
**08.04.2015 Patentblatt 2015/15**

(51) Int Cl.:
***C07C 69/704*** *(2006.01)* ***C08K 5/00*** *(2006.01)*

(21) Anmeldenummer: **14182782.4**

(22) Anmeldetag: **05.05.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **03.06.2008 DE 102008002168**

(62) Dokumentnummer(n) der früheren Anmeldung(en) nach Art. 76 EPÜ:
**09757374.5 / 2 297 083**

(71) Anmelder: **Evonik Degussa GmbH**
**45128 Essen (DE)**

(72) Erfinder:
- **Grass, Michael**
  **45721 Haltern am See (DE)**
- **Buchholz, Stefan**
  **63456 Hanau (DE)**
- **Büschken, Wilfried**
  **45721 Haltern am See (DE)**

Bemerkungen:
Diese Anmeldung ist am 29-08-2014 als Teilanmeldung zu der unter INID-Code 62 erwähnten Anmeldung eingereicht worden.

(54) **Zitronensäureestergemische und ihre Verwendung**

(57) Gegenstand der vorliegenden Erfindungen sind Gemische von Zitronensäureestern der Formel **I**,

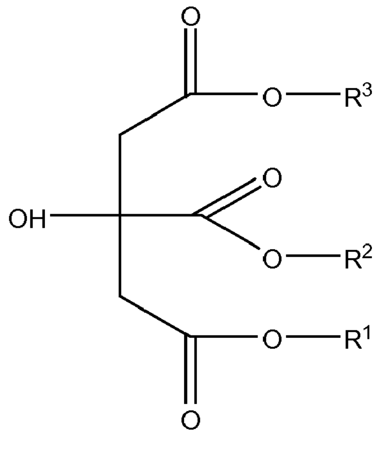

**I**

worin $R^1$, $R^2$ und $R^3$ jeweils aliphatische $C_5$- oder $C_9$-Reste sind, dadurch gekennzeichnet, dass im Gemisch die durchschnittliche Kettenlänge der aliphatischen Reste im Bereich zwischen größer 5 und 7 und der durchschnittliche Verzweigungsgrad der aliphatischen $C_9$-Reste im Bereich zwischen 0,9 und 2,2 liegt.

Ebenso Gegenstand der vorliegenden Erfindung ist die Verwendung des erfindungsgemäßen Zitronensäureestergemisches als Weichmacher in Kunststofifzusammensetzungen.

Außerdem ist Gegenstand der vorliegenden Erfindung eine Zusammensetzung, enthaltend das erfindungsgemäße Zitronensäureestergemisch in Mischung mit weiteren Komponenten, enthaltend Weichmacher aus der Gruppe der Alkylester der aromatischen Polycarbonsäuren, der Cyclohexanpolycarbonsäuren, der Benzoesäure oder der Adipinsäure sowie die Verwendung einer solchen Weichmacherzusammensetzung in Kunststoffzusammensetzungen, Klebstoffen, Dichtungsmassen, Lacken, Farben, Plastisolen oder Tinten. Aus den erfindungsgemäßen Weichmachern hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen.

EP 2 857 383 A1

## Beschreibung

Die vorliegende Erfindung betrifft Gemische aus Zitronensäureestern, die Pentylcitrate, Nonylcitrate und Nonylpentylcitrate enthalten sowie ihre Herstellung und ihre Verwendung als Weichmacher für Kunststoffe.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Diisononylphthalat (DINP) und Diisodecylphthalat (DIDP) Verwendung finden.

Auf Grund von Diskussionen um reproduktionstoxische Effekte, die bereits in einigen Fällen zu einer verstärkten gefahrstoffrechtlichen Kennzeichnung und im Falle von Kleinkinderspielzeug auch zu Verwendungsbeschränkungen geführt haben, muss davon ausgegangen werden, dass die Verwendung dieser Phthalate künftig, insbesondere in sensiblen Anwendungen, wie Lebensmittelverpackungen und Medizinalanwendungen, deutlich zurückgehen wird. Darüber hinaus wird speziell auch in Anwendungen für den Innenraum, also zum Beispiel bei Bodenbelägen, Tapeten und Kunstledern, von verschiedenen Umweltverbänden eine generelle Substitution von Phthalaten gefordert.

Es besteht daher Bedarf nach nicht kennzeichnungspflichtigen Weichmachern, die als Ersatz für DEHP aber auch DINP Verwendung finden können und die aus Rohstoffen hergestellt werden, die weltweit in großen Mengen verfügbar sind.

Eine denkbare Alternative ist die Verwendung von Weichmachern auf Basis von Zitronensäure, vor allem auch, weil diese aus nachwachsenden Rohstoffen zugänglich ist. Es ist bekannt, Zitronensäureester mit drei gleichen Alkylresten, wie beispielsweise Tri-n-butylcitrat oder Tri-2-ethylhexylcitrat einzusetzen (z. B. Gächter/Müller, Kunststoffadditive, 3.Ausgabe 1990, Carl Hanser-Verlag, Seiten 417 - 418). In DE 10 2006 026 624 werden Zitronensäureester auf Basis von primären $C_5$-Alkoholen beschrieben. Häufig werden diese Ester noch derivatisiert, indem die freie Hydroxylgruppe des Zitronensäuretriesters mit einer Carbonsäure oder einem Carbonsäurederivat, beispielsweise mit dem Anhydrid, acetyliert wird. Der bisher bekannteste Weichmacher auf Zitronensäurebasis ist das Acetyl-tri-n-butylcitrat (ATBC), ein Stoff, der auch diverse Zulassungen in sensiblen Anwendungen aufweist.

Entsprechende Acylierungsverfahren sind beispielsweise aus DE 1 099 523 bekannt. Aus DE 35 20 750 sind auch solche Zitronensäureestergemische bzw. Gemische ihrer Acetylderivate mit unterschiedlichen Alkylreste bekannt, beispielsweise ein Estergemisch, das n-Hexyl-, n-Octyl und n-Decyl-Reste aufweist.

Zitronensäureester bzw. Gemische von Zitronensäureester, die als Weichmacher verwendet werden, sollen mehrere Forderungen erfüllen: Ihre Flüchtigkeit darf nur gering sein, sodass es bei der Verarbeitung nur zu geringen Weichmacherverlusten und zu einer geringen Belastung der Umwelt kommt. Hier ist zum Beispiel das ATBC nicht optimal. Die Verträglichkeit der Weichmacher mit dem weich zu machenden Kunststoff muss groß genug sein, damit auch eine hohe Menge davon in den Kunststoff eingearbeitet werden kann und es selbst bei längerem Gebrauch zu keinem nennenswerten Ausschwitzen des Weichmachers aus dem Kunststoff kommt. Sie müssen in Mischungen mit dem Kunststoff gute Verarbeitungseigenschaften bewirken. Insbesondere muss gewährleistet sein, dass für das große Anwendungsgebiet der Plastisolverarbeitung eine ausreichende Eignung besteht. Für den Fall, dass mit einem Zitronensäureester ein bestimmter Weichmacher (zum Beispiel DINP) ersetzt werden soll, wären möglichst ähnliche Eigenschaften wünschenswert, da dies den Umstellungsaufwand in den Rezepturen und den Prozessparametern minimiert. Hier wären als wichtigste Eigenschaften die Plastisolviskosität, die Gelierung und die Effizienz (Weichmachermenge zur Erzielung einer bestimmten Weichheit) zu nennen.

Darüber hinaus sollen die Zitronensäureester bzw. ihre Gemische aus leicht verfügbaren und preiswerten Stoffen hergestellt werden können.

In EP 1 256 566 wird ein Weichmacher beschrieben, der aus einem Gemisch von Tri-n-butylcitrat, Di-n-butyl-2-ethylhexylcitrat, n-Butyl-di-2-ethylhexylcitrat und Tri-2-ethylhexylcitrat besteht. Dieser Weichmacher zeichnet sich durch geringe Flüchtigkeit und gute Verträglichkeit mit PVC aus. Weitere positive Merkmale sind nicht offenlegt.

In WO 2007/038489 werden als Weichmacher ebenfalls Citratgemische mit zwei unterschiedlichen Alkylresten, die sich um mindestens 4 C-Atome unterscheiden, offengelegt. Diese Citratgemische sollen eine relative geringe Flüchtigkeit aufweisen und im weichgemachten PVC gute anwendungstechnische Eigenschaften bewirken. Im Beispiel wird ein Citratgemisch durch Veresterung von Zitronensäure mit n-Butanol und Isononanol hergestellt, das anschließend acetyliert wird. Es handelt sich also um ein Gemisch, das aus Acetyl-tri-n-butylcitrat, Acetyl-di-n-butyl-isononylcitrat, Acetyl-n-butyl-di-isononylcitrat und Acetyl-tri-isononylcitrat besteht.

In EP 1 063257 B1 werden Zitronensäureestergemische als Weichmacher für PVC beansprucht, deren Alkoholkomponente eine Mischung von verschiedenen Alkoholen mit einem Kettenlängenbereich von $C_5$ bis $C_{12}$ umfasst. Es ist daraus nicht zu entnehmen, ob die Zitronensäure mit zwei oder mehreren Alkoholen verestert wird, ob die Alkohole gleiche oder unterschiedliche Anzahl von C-Atomen aufweisen und ob es sich um primäre, sekundäre, tertiäre Alkohole oder Gemische davon handelt. In Beispiel 1 wird ein Zitronensäureestergemisch durch Umsetzung von Zitronensäure mit Alkoholen mit einem Kettenlängenbereich von $C_6$ bis $C_{10}$ hergestellt. Diese Aussage ist vieldeutig. Alkohole mit einer

Kettenlänge von $C_6$ bis $C_{10}$ kann bedeuten, dass Alkohole jeweils mit 6, 7, 8, 9 und 10 C-Atomen vorhanden sind, es kann jedoch auch bedeuten zwei oder mehrere isomere Alkohole oder zwei oder mehrere Alkohole mit unterschiedlicher C-Zahl oder isomere Alkohole zusammen mit Alkoholen anderer C-Zahl. Weiterhin ist das molare Verhältnis der Alkohole zueinander und die Art der Alkohole (primär, sekundär, tertiär) nicht angegeben. Mit anderen Worten, es sind beliebig viele Citratestergemische mit unterschiedlichen Eigenschaften möglich. Es wird keinem definierten Citratestergemisch ein Eigenschaftsprofil zugeordnet.

Da die bekannten Zitronensäureester und deren Gemische nicht in allen Punkten dem gewünschten Anforderungsprofil entsprechen, bestand die Aufgabe auf Basis von Zitronensäure einen Weichmacher mit verbesserten Eigenschaften zu entwickeln.

Dieser Weichmacher sollte in der Lage sein, den bisherigen Standardweichmacher DINP mit möglichst geringem Aufwand ersetzen zu können. Zudem sollte der Weichmacher keine Komponenten aufweisen, die nach der Definition des Ausschusses zur gesundheitlichen Bewertung von Bauprodukten (AGBB, zum Beispiel unter http://www.umweltbundesamt.de/bauprodukte/agbb.htm) als SVOC-Komponenten (schwerflüchtige organische Verbindungen) mit einem Retentionsbereich auf einer unpolaren Gaschromatographie-Säule zwischen $C_{16}$ und $C_{22}$, einzustufen wären (s. Beispiel 7).

Seit einigen Jahren gelten in Europa verschärfte Bestimmungen zur Zulassung von Bauprodukten in Aufenthaltsräumen. Praktisch allen nationalen Standards gemeinsam ist die Festsetzung eines Maximalwerts an emittiertem VOC nach einer gewissen Lagerzeit in einer Emissionsprüfkammer. Lediglich in Deutschland gelten zusätzlich strenge Grenzwerte für schwerflüchtige Verbindungen (SVOC's). Das Fehlen dieser Verbindungen lässt dem Verarbeiter mehr Freiräume in der Formulierung. Somit können diese erfindungsgemäßen Ester zur Herstellung von Artikeln für den Innenraum verwendet werden. Weiterhin ist die Flüchtigkeit der erfindungsgemäßen Zitronensäureestergemische geringer als die von Butyl/2-Ethylhexyl, Butyl/Isononyl- und Butyl/Isodecylcitraten mit gleicher durchschnittlicher Kettenlänge der Alkylgruppen (s. Beispiel 6). Insbesondere aus Kostengründen sollte die Hydroxylgruppe nicht acyliert sein.

Überraschenderweise wurde gefunden, dass Gemische von Zitronensäurestern, deren Hydroxylgruppe nicht acetyliert ist, deren am Sauerstoff der Carboxylgruppe gebundenen Alkylreste ausschließlich $C_5$- oder $C_9$-Reste sind, und deren durchschnittliche Kettenlänge im Bereich zwischen größer 5 und 7 liegt, wobei der durchschnittliche Verzweigungsgrad der aliphatischen $C_9$-Reste im Bereich zwischen 0,9 und 2,2 liegt, die gewünschten Anforderungen erfüllen.

Gegenstand der vorliegenden Erfindungen sind Gemische von Zitronensäureestern der Formel I,

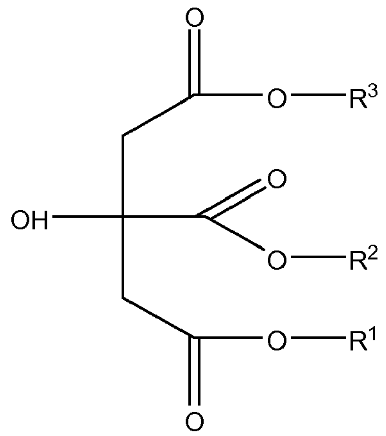

**I**

worin $R^1$, $R^2$ und $R^3$ jeweils aliphatische $C_5$- oder $C_9$-Reste sind, dadurch gekennzeichnet, dass im Gemisch die durchschnittliche Kettenlänge der aliphatischen Reste im Bereich zwischen größer 5 und 7 und der durchschnittliche Verzweigungsgrad der aliphatischen $C_9$-Reste im Bereich zwischen 0,9 und 2,2 liegt.

Diese Gemische von Zitronensäureestern schließen die jeweils auf Basis der eingesetzten aliphatischen $C_5$- und $C_9$-Alkohole gemischten Zitronensäureester, wie z. B. ein Dinonyl-pentyl-Citrat, mit ein.

Ebenso Gegenstand der vorliegenden Erfindung ist die Verwendung des erfindungsgemäßen Zitronensäureestergemisches als Weichmacher in Kunststoffzusammensetzungen.

Außerdem ist Gegenstand der vorliegenden Erfindung eine Zusammensetzung, enthaltend das erfindungsgemäße Zitronensäureestergemisch in Mischung mit weiteren Komponenten, enthaltend Weichmacher aus der Gruppe der Alkylester der aromatischen Polycarbonsäuren, der Cyclohexanpolycarbonsäuren, der Benzoesäure oder der Adipinsäure sowie die Verwendung einer solchen Weichmacherzusammensetzung in Kunststoffzusammensetzungen, Klebstoffen, Dichtungsmassen, Lacken, Farben, Plastisolen oder Tinten. Aus den erfindungsgemäßen Weichmachern

hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen.

Die erfindungsgemäßen Estergemische besitzen hohe Verträglichkeiten mit Kunststoffen und weisen gute anwendungstechnische Eigenschaften auf. In einer besonderen Ausführungsform sind die erfindungsgemäßen Zitronensäureestergemische in der Lage, den derzeit in Europa wichtigsten Weichmacher DINP ohne große Rezepturänderungen zu ersetzen, da die wichtigsten Eigenschaften:

- Plastisolviskositäten,
- Gelierung und
- weichmachende Wirkung

fast identisch sind (s. Beispiele 3, 4 und 5). Darüber hinaus ist in Plastisolen der Anstieg der Viskositäten mit der Zeit (Alterung) deutlich geringer als mit anderen Citraten.

Zur Charakterisierung des Zitronensäureestergemisch der allgemeinen Formel I

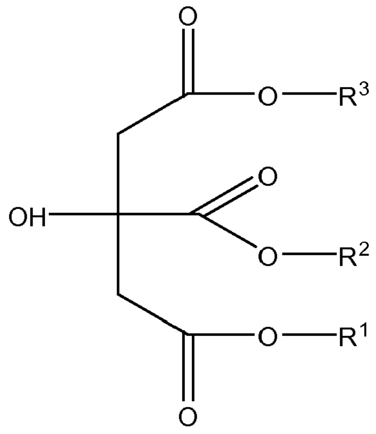

I

wird die durchschnittliche Kettenlänge bzw. die durchschnittliche Anzahl der C-Atome der am Sauerstoff der Carboxylatgruppen gebunden aliphatischen $C_5$-oder $C_9$-Reste $R^1$, $R^2$ und $R^3$ herangezogen. In der vorliegenden Erfindung liegt die durchschnittliche Anzahl der C-Atome $C_d$ der am Sauerstoff der Carboxylatgruppe gebundenen Alkylreste $R^1$, $R^2$ und $R^3$ im Bereich von größer 5 bis 7, insbesondere im Bereich von 5,5 bis 6,8.

Im Fall eines gemischten Zitronensäureesters aus 2 Alkoholen mit a Molen eines Alkohols mit x C-Atomen und b Molen eines Alkohols mit y C-Atomen errechnet sich die durchschnittliche Kettenlänge als durchschnittliche Anzahl der C-Atome $C_d$ der Alkylseitenkette gemäß:

- $C_d = (a*x + b*y) / (a+b)$

Wenn $n_1$ die Stoffmenge der $C_5$-Reste in einer bestimmten Menge des Zitronensäureesters und $n_2$ die entsprechende Stoffmenge an $C_9$-Resten in der gleichen Menge ist, berechnet man die durchschnittliche Anzahl der C-Atome $C_d$ wie folgt:

- $C_d = (5 n_1 + 9 n_2)/ (n_1 + n_2)$

Mit $v = n_1/n_2$ für das Verhältnis der Stoffmengen von $C_5$-Resten zu $C_9$-Resten, gilt für die durchschnittliche Kettenlänge der Alkylreste bzw. für $C_d$:

- $C_d = (5v + 9)/ (v + 1)$

Liegen beispielsweise $C_5$-Reste und $C_9$-Reste in äquimolaren Mengen vor, berechnet sich eine durchschnittliche Kettenlänge der Alkylreste bzw. ein Wert für $C_d$ von 7.

Erfindungsgemäß können die $C_5$-Reste folgende Strukturen aufweisen:

n-Pentyl (- $CH_2$-$CH_2$-$CH_2$-$CH_2$-$CH_3$)
2-Methylbutyl (- $CH_2$-$CH(CH_3)$-$CH_2$-$CH_3$)
3-Methylbutyl ( - $CH_2$-$CH_2$-$CH(CH_3)$-$CH_3$)
2, 2-Dimethylpropyl (- $CH_2$-$C(CH_3)_2$-$CH_3$)

Bevorzugt enthalten die erfindungsgemäßen Zitronensäureestergemische n-Pentyl- und/oder 3-Methylbutylreste. Insbesondere ist der Anteil der Summe aus n-Pentyl- und 3-Methylbutylreste an der Gesamtheit der Pentylreste größer als 90 %, ganz besonders größer als 95 %. Der Anteil an 2,2-Dimethylpropylrest liegt vorzugsweise unter 2 %, ganz besonders bevorzugt unter 1 %. Besonders bevorzugt ist der Gehalt an 3-Methylbutanol an der Gesamtheit der Pentylreste größer als 70 %, insbesondere größer als 80 %, ganz besonders bevorzugt größer als 90%.

Die $C_9$-Reste (-$CH_2$-$C_8H_{17}$) im erfindungsgemäßen Estergemisch können ebenfalls unterschiedliche Strukturen aufweisen, wobei die Gruppe -$C_8H_{17}$ des $C_9$-Rest linear, einfach verzweigt, zweifach oder mehrfach verzweigt sein kann. Vorzugsweise enthält das Estergemisch eine Vielzahl von isomeren $C_9$-Resten, deren durchschnittlicher Verzweigungsgrad vorzugsweise zwischen 0,9 bis 2,2 und besonders bevorzugt zwischen 1,0 und 2,0 liegen kann.

Die Ermittlung des durchschnittlichen Verzweigungsgrades der $C_9$-Reste in den Zitronensäureestergemischen kann durch $^1$H- oder $^{13}$C-NMR spektroskopische Verfahren bestimmt werden.

Die erfindungsgemäßen Zitronensäureestergemische I können auf folgenden Wegen hergestellt werden:

a) durch Veresterung von Zitronensäure, Zitronensäure-Monohydrat oder Zitronensäure-Anhydrid mit Pentanolen und Nonanolen
b) Umesterung von Zitronensäureester, beispielsweise Trimethylcitrat oder Triethylcitrat, mit Pentanolen und Nonanolen
c) Umesterung eines Zitronensäurepentylesters oder -Gemischs mit einem Zitronensäurenonylesters oder -Gemisches
d) Mischung der jeweils gemischten Zitronensäureester auf Basis der eingesetzten aliphatischen $C_5$- und $C_9$-Alkohole
e) Mischung von Trinonylcitraten mit Tripentylcitraten

Bevorzugt wird das erfindungsgemäße Gemisch nach dem Weg a) hergestellt. Eine weitere bevorzugte Herstellungsmethode ist die Kombination aus Weg a) und b). Dabei wird die Zitronensäure oder eines ihrer Anhydride mit Pentanol oder einem Pentanolgemisch zu Pentylester(n) verestert, wobei überschüssiges Pentanol als Schleppmittel für Wasser dient. Der entstandene Tripentylester wird anschließend mit der berechneten Menge an Nonanol umgeestert. Optional kann die Zusammensetzung der Zitronensäureestergemische destillativ geändert werden.

Bei der Veresterung der Zitronensäure oder eines Zitronensäurehydrat mit dem entsprechenden Alkohol oder Alkoholgemisch wird die Alkoholkomponente im stöchiometrischen Überschuss eingesetzt. Die Alkoholkomponente ist Reaktionspartner und Schleppmittel zur Abtrennung des Reaktionswassers und gegebenenfalls des mit der Zitronensäure eingebrachten Wassers. Der stöchiometrische Überschuss beträgt vorzugsweise 5 bis 50 %, insbesondere 10 bis 40 %, besonders bevorzugt 15 bis 35 %.

Die Veresterung wird vorzugsweise in Gegenwart eines Veresterungskatalysators durchgeführt. Als Katalysatoren können prinzipiell Säuren, wie beispielsweise Schwefelsäure, Methansulfonsäure oder p-Toluolsulfonsäure, andere Mineralsäuren oder Metalle oder deren Verbindungen eingesetzt werden. Geeignet sind z. B. Zinn, Titan, Zirkonium, die als feinverteilte Metalle oder zweckmäßig in Form ihrer Salze, Oxide oder in Form von löslichen organischen Verbindung verwendet werden können. Allerdings sind die Metallkatalysatoren im Vergleich zu den Protonensäuren Hochtemperaturkatalysatoren, die ihre volle Aktivität oft erst bei Temperaturen oberhalb von 180 °C erreichen.

Die Katalysatorkonzentration kann abhängig von der Art des Katalysators in weiten Bereichen variiert werden. Für Protonensäuren sind Konzentrationen von 0,05 bis 2 Massen-%, bevorzugt von 0,1 bis 1 Massen-%, besonders bevorzugt von 0,15 bis 0,5 Massen-%, üblich. Höhere Säurekonzentrationen erhöhen zwar die Reaktionsgeschwindigkeit, können jedoch Nebenreaktionen begünstigen. Um beispielsweise die Wasserabspaltung aus Zitronensäure oder deren Partialester oder Vollester zu vermeiden, ist es zweckmäßig, die Veresterung im Temperaturbereich von 120 °C bis 180 °C, bevorzugt 130 °C bis 170 °C und ganz besonders bevorzugt bei 155 °C bis 165 °C durchzuführen.

Die einzusetzenden aliphatischen $C_9$-Alkohole sind in verschiedener Zusammensetzung kommerziell am Markt erhältlich. Im Rahmen dieser Erfindung können auch Nonanolgemische eingesetzt werden, die neben $C_9$- auch Mengen an $C_8$- sowie $C_{10}$-Alkoholen enthalten.

Bei der Veresterung wird als $C_9$-Alkoholkomponente bevorzugt ein $C_9$-Alkoholgemisch verwendet, das durch

Hydroformylierung eines $C_8$-Olefingemisches, hergestellt durch Oligomerisierung von linearen Butenen nach dem Octol-Prozess an einem Nickel enthaltenden Katalysator, und anschließende Hydrierung gewonnen wird.

Die erfindungsgemäßen Zitronensäureestergemische können als Weichmacher, insbesondere in Kunststoffzusammensetzungen, Klebstoffen, Dichtungsmassen, Lacken, Farben, Plastisolen, Kunstledern, Fußbodenbelägen, Unterbodenschutz, beschichteten Geweben, Tapeten oder Tinten verwendet werden. Vorzugsweise können die erfindungsgemäßen Weichmacher in Profilen, Dichtungen, Lebensmittelverpackungen, Folien, Spielzeugen, Medizinalartikeln, Dachbahnen, Kunstledern, Fußbodenbelägen, Unterbodenschutz, beschichteten Geweben, Tapeten, Kabeln und Drahtummantelungen, besonders bevorzugt in Lebensmittelverpackungen, Spielzeugen, Medizinalartikeln, Tapeten und Fußbodenbelägen verwendet werden.

Unter Verwendung der erfindungsgemäßen Zitronensäureestergemische sind insbesondere erfindungsgemäße Zusammensetzungen erhältlich, die das Zitronensäureestergemisch enthalten.

Solche Zusammensetzungen können das erfindungsgemäße Zitronensäureestergemisch alleine oder in Mischungen mit anderen Weichmachern aufweisen. Falls die erfindungsgemäßen Zusammensetzungen das erfindungsgemäße Zitronensäureestergemisch im Gemisch mit anderen Weichmachern aufweisen, so können die anderen Weichmacher vorzugsweise aus der Gruppe der Phthalsäuredialkylester, bevorzugt mit 4 bis 13 C-Atomen in der Alkylkette; Trimellitsäuretrialkylester, bevorzugt mit 4 bis 10 C-Atomen in der Seitenkette; Adipinsäuredialkylester und bevorzugt Terephthalsäuredialkylester jeweils bevorzugt mit 4 bis 10 C-Atomen in der Seitenkette; 1,2-Cyclohexandisäurealkylestern, 1,3-Cyclohexandisäurealkylestern und 1,4-Cyclohexandisäurealkylestern, bevorzugt 1,2-Cyclohexandisäurealkylestern, jeweils bevorzugt mit Alkyl = Alkylrest mit 4 bis 10 Kohlenstoffatomen in der Seitenkette; Dibenzoesäurester von Glykolen, bevorzugt mit Di- oder Triethylenglykol wie auch Di- oder Tripropylenglykol; Alkylsulfonsäurester von Phenol mit vorzugsweise einem Alkylrest, der 8 bis 22 C-Atome enthält; Polymerweichmacher, Glycerinester und Benzoesäurealkylester, vorzugsweise mit 7 bis 13 C-Atomen in der Alkylkette, ausgewählt sein.

In allen Fällen können die Alkylreste linear oder verzweigt sowie gleich oder verschieden sein. Besonders bevorzugt weist die Zusammensetzung neben Zitronensäureestergemischen insbesondere einen Benzoesäurealkylester mit Alkyl = Alkylrest mit 7 bis 13 Kohlenstoffatomen, vorzugsweise Benzoesäureisononylester, Benzoesäurenonylester, Benzoesäureisodecylester, Benzoesäurepropylheptylester oder Benzoesäuredecylester auf. Der Anteil an erfindungsgemäßen Zitronensäureestergemischen in dem Gemisch mit anderen Weichmachern beträgt vorzugsweise 15 bis 90 %, besonders bevorzugt 20 bis 80 % und ganz besonders bevorzugt 30 bis 70 %, wobei sich die Massenanteile aller vorhandenen Weichmacher zu 100 % addieren.

Die genannten Zusammensetzungen aus Zitronensäureestergemischen und anderen Weichmachern können als Weichmacherzusammensetzung in Kunststoffzusammensetzungen, Klebstoffen, Dichtungsmassen, Lacken, Farben, Plastisolen oder Tinten verwendet werden. Aus den erfindungsgemäßen Weichmacherzusammensetzungen hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen. Bevorzugt sind aus dieser Gruppe Lebensmittelverpackungen, Spielzeug, Medizinalartikel, Tapeten, beschichtete Gewebe, Kunstleder und Fußbodenbeläge zu nennen.

Die erfindungsgemäßen Zusammensetzungen, die ein Zitronensäureestergemisch enthalten, können ein Polymer, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalcohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxylbuttersäure (PHB), Polyhydroxylvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten aufweisen. Vorzugsweise weisen die erfindungsgemäßen Zusammensetzungen PVC oder Homo- oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Bevorzugt enthält die erfindungsgemäße Zusammensetzung als PVC-Typ Suspensions-, Masse-, Mikrosuspensions- oder Emulsions-PVC. Bezogen auf 100 Massenteile Polymer enthalten die erfindungsgemäßen Zusammensetzungen vorzugsweise von 5 bis 200, bevorzugt von 10 bis 150 Massenteile an Weichmacher.

Die erfindungsgemäßen Zusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie

beispielsweise epoxidiertes Sojabohnenöl, Gleitmittel, Treibmittel, Kicker, Antioxidanzien oder Biozide.

Die genannten Polymere aufweisenden Zusammensetzungen können als Kunststoffzusammensetzungen, Klebstoffe, Dichtungsmassen, Lacke, Farben, Plastisole, Kunstleder, Fußbodenbeläge, Unterbodenschutz, Gewebebeschichtungen, Tapeten oder Tinten oder zu deren Herstellung verwendet werden. Die genannten Zusammensetzungen können insbesondere Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen sein. Bevorzugt sind die Zusammensetzungen Lebensmittelverpackungen, Spielzeug, Medizinalartikel, Tapeten und Fußbodenbeläge zu nennen.

Die folgenden Beispiele sollen die Erfindung erläutern, ohne diese darauf zu beschränken.

## Beispiele

Beispiel 1: Synthese der Zitronensäureester

Gemäß der folgenden Vorschrift wurde jeweils Zitronensäure-Monohydrat mit den in Tabelle 1 aufgeführten Mischungen eines kürzerkettigen (A1) und eines längerkettigen Alkohols (A2) verestert. Die Alkoholmischungen wurden vorab hergestellt und stellen jeweils bezogen auf die Zitronensäure einen Überschuss von 33 % dar.

1050 g Zitronensäure-1-hydrat ( 5 Mol) und zunächst 800 g Alkoholgemisch gemäß Tabelle 1 wurden in einem 4 Liter Destillationskolben vorgelegt. Der Kolbeninhalt und die Veresterungsapparatur wurden über ein Tauchrohr zunächst 30 Minuten mit Stickstoff gespült. Der Rührer wurde mit geringer Drehzahl eingeschaltet und der Ansatz langsam aufgeheizt. Ab ca. 115 °C fiel Kristallwasser aus der Säure an, das über einen Wasserabscheider abgenommen wurde. Bei 145 °C wurden 3,15 g Schwefelsäure (0,3 Massen-% bezogen auf die Carbonsäure) verdünnt in 50ml Alkoholgemisch, über einen angebrachten Tropftrichter, der vor der Zugabe ca. 5 Minuten mit Stickstoff gespült wurde, zugetropft. Während der Zugabe blieb der Tropftrichter mit Stickstoff überlagert.

Nach Erreichen der Reaktionstemperatur von 160 °C wurde die restliche Alkoholmenge langsam zugegeben. Dabei wurde darauf geachtet, dass die Temperatur konstant blieb. Das anfallende Reaktionswasser wurde regelmäßig abgenommen. Nach vollständiger Zugabe des Alkohols wurde bei konstanter Temperatur der Rückfluss über Zugabe von Toluol aufrecht erhalten. Nach 5-7 Stunden wurde die Reaktion beendet, da die Säurezahl auf unter 1 mg KOH/g abgesunken war. Danach wurde der Ansatz unter Fortsetzung des Stickstoffstromes abgekühlt. Der Reaktionsaustrag wurde dann in einen 4 L Reaktionskolben, ausgerüstet mit Flügelrührer, Tauchrohr mit aufgesetztem Tropftrichter und Thermometer an einer Claisenbrücke, umgefüllt. Auch hier wurde darauf geachtet, dass die Inertgasatmosphäre bestehen blieb. Anschließend wurde weitere 15 Minuten mit Stickstoff gespült, dann wurde Vakuum angelegt und nach 15 Minuten wurde die Heizung eingeschaltet. Unter maximalem Vakuum wurden bis 160 °C das zugegebene Toluol und der Alkoholüberschuss abdestilliert und dann vom Rückstand die Säurezahl nach DIN EN ISO 2114 bestimmt und mit der zehnfachen stöchiometrischen Natronlauge-Menge (5 Massen-% in Wasser) neutralisiert. Hierzu wurde die Laugemenge bei 80 °C unter Normaldruck, über den Tropftrichter, langsam zugetropft. Nach vollständiger Zugabe wurde 30 Minuten bei 80 °C gerührt. Danach wurde der Rührer abgestellt. Nach einer Absetzzeit von 30 Minuten wurde die wässrige Phase abgelassen.

In den Rohester wurde anschließend 5%ige wässrige Kochsalzlösung gegeben (25 Massen-% bezogen auf den Reaktorinhalt) und es wurde 15 Minuten bei 80 °C gerührt. Danach wurde der Rührer abgestellt. Nach einer Absetzzeit von 30 Minuten wurde die wässrige Phase abgelassen.

Dem neutralisierten Ester wurden 2 % Aktivkohle (CAP Super, Fa. Norit), bezogen auf den Reaktorinhalt zugesetzt, der Reaktor evakuiert und auf 140 °C aufgeheizt. Über ein Tauchrohr wurde Stickstoff in das Produkt eingeleitet. Über den Stickstoffstrom wurde das Vakuum auf 40 mbar eingestellt und 30 Minuten gestrippt.

Anschließend wurde die Apparatur auf 80 °C abgekühlt.

Gefiltert wurde über eine Saugflasche mit Nutsche, wobei die Nutsche mit Filterpapier belegt war, auf dem zuvor ein Filterkuchen aus Filterhilfsmittel vorgepresst wurde.

Die Reinheit der so hergestellten Ester wurde anschließend per Gaschromatographie bestimmt. Sie lag in allen Fällen oberhalb von 99,7 %.

Tabelle 1:

| Versuch | A1 | Menge A1 in g (mol) | A2 | Menge A2 in g (mol) | durchschnittliche C-Zahl $C_d$ |
|---|---|---|---|---|---|
| 1 (erfindungsgemäß) | 3-Methylbutanol (Aldrich) | 1408 (16) | Isononanol (Evonik Oxeno) | 576 (4) | 5,8 |

(fortgesetzt)

| Versuch | A1 | Menge A1 in g (mol) | A2 | Menge A2 in g (mol) | durchschnittliche C-Zahl $C_d$ |
|---|---|---|---|---|---|
| 2 (erfindungsgemäß) | 3-Methylbutanol (Aldrich) | 1179 (13,4) | Isononanol (Evonik Oxeno) | 950 (6,6) | 6,3 |
| 3 | n-Butanol (OXEA) | 814 (11) | 2-Ethylhexanol (OXEA) | 1170 (9) | 5,8 |
| 4 | n-Butanol (OXEA) | 947 (12,8) | Isononanol (Evonik Oxeno) | 1037 (7,2) | 5,8 |
| 5 | n-Butanol (OXEA) | 1036 (14) | Isodecanol (Exxal 10 der Fa. ExxonMobil) | 924 (6) | 5,8 |

**Beispiel 2: Herstellung von Plastisolen**

Die verwendeten Einwaagen der Komponenten für die verschiedenen Plastisole sind der nachfolgenden Tabelle 2 zu entnehmen.

Tabelle 2: Rezepturen [Alle Angaben in phr (= Massenteile pro 100 Massenteile PVC)]

| **Plastisolrezeptur** | **1** | **2** | **3** | **4** | **5** | **6** |
|---|---|---|---|---|---|---|
| Vestolit B 7021 (Vestolit GmbH) | 100 | 100 | 100 | 100 | 100 | 100 |
| Vestinol 9 (DINP der Evonik Oxeno GmbH) | 50 | | | | | |
| Citrat 1 (erfindungsgemäß) | | 50 | | | | |
| Citrat 2 (erfindungsgemäß) | | | 50 | | | |
| Citrat 3 (Vergleichsbeispiel) | | | | 50 | | |
| Citrat 4 (Vergleichsbeispiel) | | | | | 50 | |
| Citrat 5 (Vergleichsbeispiel) | | | | | | 50 |
| Epoxidiertes Sojabohnenöl (Drapex 39, Fa. Chemtura) | 3 | 3 | 3 | 3 | 3 | 3 |
| OBS 1100, Stabilisator (Fa. Chemtura) | 0,6 | 0,6 | 0,6 | 0,6 | 0,6 | 0,6 |
| Mark CH 302, Costabilisator (Fa. Chemtura) | 1,8 | 1,8 | 1,8 | 1,8 | 1,8 | 1,8 |

Die Weichmacher wurden vor der Zugabe auf 25 °C temperiert. Zuerst wurden die flüssigen Bestandteile und dann die pulverförmigen in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Vor dem Eintauchen des Rührers in die Mischung wurde die Drehzahl auf 1800 Umdrehungen pro Minute eingestellt. Nach dem Einschalten des Rührers wurde so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

**Beispiel 3: Messung der Plastisolviskosität**

Die Messung der Viskositäten der in Beispiel 2 hergestellten Plastisole wurden mit einem Rheometer Physica DSR 4000 (Fa. Paar-Physica), welches über die zugehörige Software US 200 gesteuert wird, wie folgt durchgeführt.

Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o. g. Software. Folgende Punkte wurden angesteuert:

Eine Vorscherung von 100 $s^{-1}$ für den Zeitraum von 60 s, bei der keine Messwerte aufgenommen wurden.

Eine Abwärtsrampe, beginnend bei 200 $s^{-1}$ bis herunter zu 0,1 $s^{-1}$, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h, 24 h und 7 Tagen durchgeführt. Zwischen diesen Zeitpunkten wurde die Paste bei 25 °C gelagert.

In der nachfolgenden Tabelle 3 sind exemplarisch für die Schergeschwindigkeit von 100 $s^{-1}$ jeweils die nach den angegebenen Lagerzeiten erhaltenen entsprechenden Viskositätswerte aufgeführt.

Tabelle 3: Plastisolviskositäten bei einer Schergeschwindigkeit von 100 $s^{-1}$

| Plastisol-Nr | Viskosität nach 2 Stunden in Pa*s | Viskosität nach 24 Stunden in Pa*s | Viskosität nach 7 Tagen in Pa*s | Prozentualer Anstieg in % |
|---|---|---|---|---|
| 1 | 6,88 | 7,15 | 7,19 | 4,5 |
| 2 | 6,20 | 6,78 | 7,0 | 13 |
| 3 | 6,67 | 7,44 | 7,19 | 7,8 |
| 4 | 5,43 | 6,17 | 6,83 | 25,7 |
| 5 | 5,32 | 5,97 | 6,57 | 23,5 |
| 6 | 5,96 | 6,71 | 7,22 | 21,1 |

Fazit: Die Plastisole aus den beiden erfindungsgemäßen Zitronensäureestergemische zeigen praktisch gleiche Viskositäten, wie das mit DINP. Die drei Citrat-Vergleichsplastisole (Plastisole 4 - 6) liegen im Niveau insgesamt etwas tiefer und zeigen darüber hinaus eine deutlich höhere Viskositätszunahme mit der Zeit. Insbesondere letzteres bedeutet für den Verarbeiter einen höheren Arbeitsaufwand. Auch aus diesem Grund sind die erfindungsgemäßen Plastisole vorzuziehen.

**Beispiel 4: Messung der Geliergeschwindigkeit**

Die Untersuchung des Gelierverhaltens der Plastisole wurde in einem Oszillationsviskosimeter der Marke Bohlin CVO (Meßsystem PP20), welches schubspannungsgesteuert betrieben wurde, vorgenommen.

Folgende Parameter wurden eingestellt:

Modus:
Temperatur-Gradient
Start-Temperatur: 25 °C
End-Temperatur: 180 °C
Heiz/Kühlrate: 2 °C/min
Temperatur nach der Messung: 25 °C
Oszillations-Frequenz: 2 Hz
Verzögerungszeit: 1 s
Wartezeit: 15 s
Kontinuierliche Oszillation: an
Automatische Schubspannungsvorgabe: an
Startschubspannung: 0,3 Pa
Soll-Deformation: 0,002
Spaltweite 0,5 mm

Durchführung der Messung:

Auf die untere Messsystemplatte wurde mit dem Spatel ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Schutzabdeckung, die auch der Wärmeisolierung dient, aufgelegt und die Messung gestartet. Bestimmt wurde die sog. komplexe Viskosität des Plastisols in Abhängigkeit von der Temperatur. Ein Einsetzen des Geliervorganges war in einem plötzlichen starken Anstieg der komplexen Viskosität zu erkennen. Je früher dieser Viskositätsanstieg einsetzte,

desto besser war die Gelierfähigkeit des Systems. Für einen Vergleich wurde aus den Kurven durch Interpolation für jedes Plastisol die Temperatur bestimmt, bei der eine komplexe Viskosität von 1000 Pa * s erreicht war.

Hierbei ergaben sich die in Tabelle 4 aufgeführten Werte:

Tabelle 4: Gelierverhalten

| Plastisol-Nummer gemäß Bespiel 2 | 1 | 2 | 3 | 4 | 5 | 6 |
|---|---|---|---|---|---|---|
| Temperatur bei Viskosität 1000 Pa * s | 92 °C | 91 °C | 94 °C | 85 °C | 86 °C | 88 °C |

Die beiden erfindungsgemäßen Zitronensäureestergemische (In den Plastisolen 2 und 3) zeigen in ihren jeweiligen Formulierungen sehr ähnliches Gelierverhalten wie das DINP.

**Beispiel 5: Messung der Shore-Härte von Gießlingen**

Die Shore-Härte A ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Umgekehrt kann bei sehr effizienten Weichmachern ein gewisser Anteil in der Rezeptur eingespart werden, was in vielen Fällen für den Verarbeiter geringere Kosten bedeutet.

Zur Bestimmung der Shore-Härten wurden die gemäß Beispiel 2 hergestellten Plastisole in kreisrunde Gießformen mit einem Durchmesser von 50 mm gegossen. Dann wurden die Plastisole in den Formen im Umlufttrockenschrank 10 min bei 200 °C geliert, nach Abkühlung entnommen und vor der Messung mindestens 16 Stunden im Normklima (23 °C; 50 % relative Feuchte) gelagert. Die Dicke der Scheiben betrug ca. 8 mm.

Die Messungen selbst wurden nach DIN 53 505 mit einem Shore-A-Messgerät der Fa. Zwick-Roell durchgeführt, der Messwert jeweils nach 3 Sekunden abgelesen. An jedem Probekörper wurden drei verschiedene Messungen an verschiedenen Stellen (nicht im Randbereich) durchgeführt und jeweils der Mittelwert notiert.

In Tabelle 5 sind die erhaltenen Messwerte aufgeführt.

Tabelle 5: Shore-Härten

| Plastisol-Nummer (s. Tabelle 2) | Alkoholmischung | Shore-Härte |
|---|---|---|
| 1 | *DINP* | 79 |
| 2 (erfindungsgemäß) | 3-Methylbutyl / Isononyl | 79 |
| 3 (erfindungsgemäß) | 3-Methylbutyl / Isononyl | 81 |
| 4 (Vergleich) | Butyl / 2-Ethylhexyl | 76 |
| 5 (Vergleich) | Butyl / Isononyl | 77 |
| 6 (Vergleich) | Butyl / Isodecyl | 79 |

Das erfindungsgemäße Zitronensäureestergemisch (Plastisol 2) zeigt die gleiche weichmachende Wirkung wie DINP.

Somit konnte gezeigt werden, dass die erfindungsgemäßen Zitronensäureestergemische den Standardweichmacher DINP insbesondere in den für die Plastisolverarbeitung wichtigsten Eigenschaften ohne Veränderung der Mengenverhältnisse von PVC und Weichmacher ersetzen können.

**Beispiel 6: Bestimmung der Flüchtigkeit der Weichmacher über Thermogravimetrie (TGA)**

Um eine Aussage über die Flüchtigkeit der Produkte zu erhalten, wurden die nach Beispiel 1 hergestellten Zitronensäureester mit Hilfe der dynamischen TGA-Methode bezüglich ihrer Masseverluste bei höheren Temperaturen verglichen.

Zu diesem Zweck wurden etwa 40 mg einer Probe unter Stickstoffatmosphäre in einem Gerät der Marke DuPont Instruments TGA 951 in einem Temperaturbereich von 20 bis 300 °C bei einer dynamischen Temperatursteigerung von 10 K/min aufgeheizt und der jeweilige Masseverlust in % bestimmt.

In Abbildung 1 sind die jeweiligen Messkurven vergleichend dargestellt.

Die beiden erfindungsgemäßen Citrate zeigen insbesondere bei den höheren Temperaturen geringere Masseverluste. Dies ist auf Grund der Tatsache, dass die mittlere Kettenlänge und somit auch die mittlere Molmasse gleich

sind, überraschend.

**Beispiel 7: Untersuchung auf SVOC-Komponenten**

Unter einem SVOC wird ein Stoff verstanden, der auf einer unpolaren GC-Säule zwischen den Retentionszeiten von $C_{16}$ und $C_{22}$ n-Paraffin eluiert.

Zur Vereinfachung wurde in allen Fällen nur die jeweils niedrigste siedende Komponente des jeweiligen Citratestergemisches, also Tri-3-methylbutylcitrat in den Fällen 1 und 2 (gemäß Tabelle 1) sowie Tri-n-butylcitrat aus den Vergleichsbeispielen 3 bis 5 (gemäß Tabelle 1) untersucht.

Diese Untersuchung erfolgte gaschromatographisch auf einer unpolaren Säule. Das Signal des Tributylcitrats erschien hier unmittelbar vor dem $C_{22}$-Paraffin-Signal, das Signal des Tri-3-methylbutylcitrates deutlich danach. Somit gilt Tributylcitrat als SVOC und Artikel die diesen Weichmacher enthalten, laufen daher Gefahr, ggf. die Maximalwerte zu überschreiten. Da Tri-3-methylbutylcitrat gemäß dieser Definition nicht als SVOC gilt, bestehen hier keine Einschränkungen zur Verwendung hieraus gefertigter Artikel in Innenräumen.

Zusammenfassend kann gesagt werden, dass die erfindungsgemäßen Citratestergemische den bisherigen Standard-Weichmacher DINP weitgehend ohne Rezepturanpassungen ersetzen könnten und im Gegensatz zu den Vergleichssubstanzen keine als SVOC zu bezeichnenden Komponenten aufweisen. Darüber hinaus ist bei den Plastisolen auf Basis der erfindungsgemäßen Citrat-Weichmacher der Viskositätsanstieg über die Zeit deutlich geringer, was einen geringeren Anpassungsbedarf für den Verarbeiter zur Folge hat.

## Patentansprüche

1. Gemische von Zitronensäureestern der Formel I,

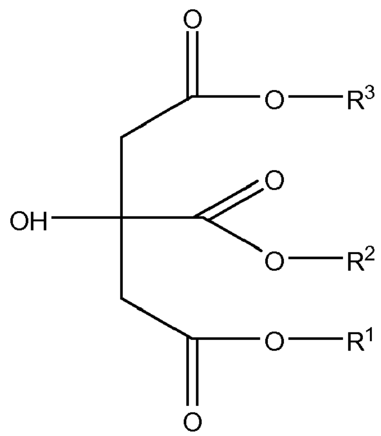

**I**

worin $R^1$, $R^2$ und $R^3$ jeweils aliphatische $C_5$- oder $C_9$-Reste sind,
**dadurch gekennzeichnet,**
**dass** im Gemisch die durchschnittliche Kettenlänge der aliphatischen Reste im Bereich zwischen größer 5 und 7 und der durchschnittliche Verzweigungsgrad der aliphatischen $C_9$-Reste im Bereich zwischen 0,9 und 2,2 liegt.

2. Gemische von Zitronensäureestern nach Anspruch 1,
**dadurch gekennzeichnet,**
**dass** Gemische von gemischten Zitronensäureestern auf Basis der eingesetzten $C_5$- und $C_9$-Alkohole nebeneinander vorliegen.

3. Gemische von Zitronensäureestern nach Anspruch 1,
**dadurch gekennzeichnet,**
**dass** Gemische von Trinonyl- und Tripentylcitraten vorliegen.

4. Gemische von Zitronensäureestern nach Anspruch 1,
**dadurch gekennzeichnet, dass** die durchschnittliche Kettenlänge der aliphatischen Reste im Bereich von 5,5 bis

6,8 liegt.

5. Gemische von Zitronensäurestern nach Anspruch 1, **dadurch gekennzeichnet,** **dass** die $C_5$-Reste mindestens zu 90 % aus n-Pentyl- und 3-Methylbutylresten bestehen.

6. Gemische von Zitronensäureestern nach Anspruch 1, **dadurch gekennzeichnet,** **dass** die $C_5$-Reste zu wenigstens 70 % aus 3-Methylbutylresten bestehen.

7. Gemische von Zitronensäuresäureestern nach Anspruch 1, **dadurch gekennzeichnet,** **dass** der durchschnittliche Verzweigungsgrad der $C_9$-Reste zwischen 1,0 und 2,0 liegt.

8. Verwendung der Gemische gemäß den Ansprüchen 1 bis 7 als Weichmacher in Kunststoffzusammensetzungen.

9. Verwendung der Gemische gemäß den Ansprüchen 1 bis 7 als Filmbildehilfsmittel in Kunststoffzusammensetzungen.

10. Weichmacherzusammensetzungen enthaltend Gemische von Zitronensäureestern gemäß den Ansprüchen 1 bis 7.

11. Weichmacherzusammensetzungen nach Anspruch 10, **dadurch gekennzeichnet,** **dass** sie weitere Weichmacher als Mischungskomponenten aus der Gruppe der Alkylester, enthaltend aromatische Polycarbonsäuren, Cyclohexanpolycarbonsäuren, Benzoesäure oder Adipinsäure sowie Diolbenzoate aus der Gruppe der Diethylenglykole, Dipropylenglykole, Triethylenglykole oder Tripropylenglykole in einem Bereich von 15 bis zu 90 Massen-% enthalten, wobei sich die Massenanteile aller verwendeten Weichmacher zu 100 Massen-% addieren.

12. Kunststoffzusammensetzungen, enthaltend Weichmacherzusammensetzungen gemäß dem Anspruch 11.

13. Kunststoffzusammensetzungen nach Anspruch 12, **dadurch gekennzeichnet,** **dass** Polyvinylchlorid (PVC) enthalten ist.

14. Kunststoffzusammensetzungen nach Anspruch 12, **dadurch gekennzeichnet,** **dass** Polyalkylmethacrylat (PAMA) enthalten ist.

15. Kunststoffzusammensetzungen nach Anspruch 12, **dadurch gekennzeichnet,** **dass** Polyvinylacetat (PVAc) enthalten ist.

16. Kunststoffzusammensetzungen nach Anspruch 12, **dadurch gekennzeichnet,** **dass** Polyvinylbutyral (PVB) enthalten ist.

17. Kunststoffzusammensetzungen nach Anspruch 12, **dadurch gekennzeichnet,** **dass** Polymilchsäure (PLA) enthalten ist.

18. Kunststoffzusammensetzungen nach Anspruch 12, **dadurch gekennzeichnet,** **dass** Polyhydroxybuttersäure (PHB) enthalten ist.

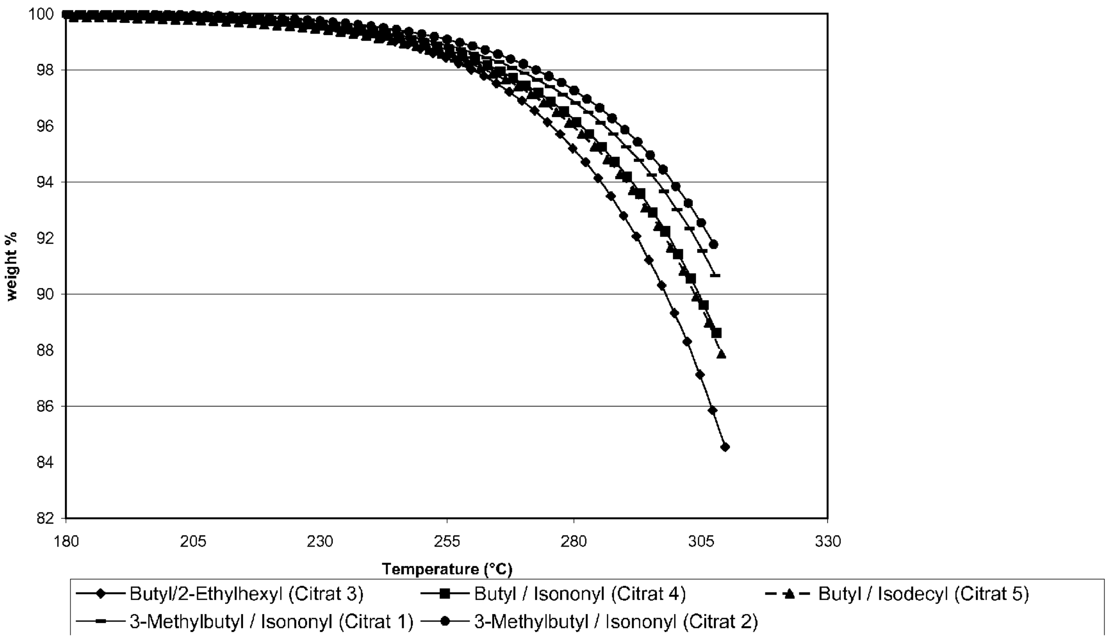
100
98
96
94
92
90
88
86
84
82
weight %
180
205
230
255
280
305
330
Temperature (°C)
Butyl/2-Ethylhexyl (Citrat 3)
Butyl / Isononyl (Citrat 4)
Butyl / Isodecyl (Citrat 5)
3-Methylbutyl / Isononyl (Citrat 1)
3-Methylbutyl / Isononyl (Citrat 2)

Abbildung 1

Europäisches Patentamt
European Patent Office
Office européen des brevets

# EUROPÄISCHER RECHERCHENBERICHT

Nummer der Anmeldung
EP 14 18 2782

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 1 256 566 A (CELANESE CHEM EUROPE GMBH [DE]) 13. November 2002 (2002-11-13)<br>* Zusammenfassung *<br>* Beispiele 1,2 *<br>* Anspruch 1 *<br>----- | 1-18 | INV.<br>C07C69/704<br>C08K5/00 |
| A,D | EP 1 864 964 A (OXENO OLEFINCHEMIE GMBH [DE]) 12. Dezember 2007 (2007-12-12)<br>* Zusammenfassung *<br>* Beispiele 1,3-7 *<br>----- | 1-18 | |
| A,D | WO 2007/038489 A (VERTELLUS PERFORMANCE MATERIAL [US]; FRAPPIER ED [US]; KENNEDY SAM [US) 5. April 2007 (2007-04-05)<br>* Zusammenfassung *<br>* Beispiel 1 *<br>* Ansprüche 6-9 *<br>----- | 1-18 | |
| A,D | EP 1 063 257 A (ERA BESCHICHTUNG GMBH & CO KG [DE]) 27. Dezember 2000 (2000-12-27)<br>* Zusammenfassung *<br>* Beispiel 1 *<br>* Anspruch 1 *<br>----- | 1-18 | RECHERCHIERTE SACHGEBIETE (IPC)<br>C07C<br>C08K |
| E<br><br><br>L | WO 2009/146991 A2 (EVONIK OXENO GMBH [DE]; GRASS MICHAEL [DE]; BUCHHOLZ STEFAN [DE]; BUES) 10. Dezember 2009 (2009-12-10)<br>* das ganze Dokument *<br>----- | 1-18 | |

Der vorliegende Recherchenbericht wurde für alle Patentansprüche erstellt

1

| Recherchenort | Abschlußdatum der Recherche | Prüfer |
|---|---|---|
| München | 2. März 2015 | Panday, Narendra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 14 18 2782

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-03-2015

| Im Recherchenbericht angeführtes Patentdokument | Datum der Veröffentlichung | Mitglied(er) der Patentfamilie | Datum der Veröffentlichung |
|---|---|---|---|
| EP 1256566 A | 13-11-2002 | DE 10122145 A1 | 28-11-2002 |
| | | EP 1256566 A2 | 13-11-2002 |
| | | KR 20020085812 A | 16-11-2002 |
| | | PL 353721 A1 | 18-11-2002 |
| | | US 2002198402 A1 | 26-12-2002 |
| EP 1864964 A | 12-12-2007 | AT 488490 T | 15-12-2010 |
| | | CA 2591251 A1 | 08-12-2007 |
| | | CN 101085736 A | 12-12-2007 |
| | | DE 102006026624 A1 | 13-12-2007 |
| | | EP 1864964 A1 | 12-12-2007 |
| | | JP 5322079 B2 | 23-10-2013 |
| | | JP 2007326859 A | 20-12-2007 |
| | | SG 138520 A1 | 28-01-2008 |
| | | TW 200815338 A | 01-04-2008 |
| | | US 2007287781 A1 | 13-12-2007 |
| WO 2007038489 A | 05-04-2007 | US 2007072988 A1 | 29-03-2007 |
| | | WO 2007038489 A2 | 05-04-2007 |
| EP 1063257 A | 27-12-2000 | AT 304033 T | 15-09-2005 |
| | | DE 19928239 A1 | 28-12-2000 |
| | | DE 50011097 D1 | 13-10-2005 |
| | | EP 1063257 A1 | 27-12-2000 |
| | | ES 2247974 T3 | 16-03-2006 |
| | | US 6534577 B1 | 18-03-2003 |
| WO 2009146991 A2 | 10-12-2009 | DE 102008002168 A1 | 10-12-2009 |
| | | EP 2297083 A2 | 23-03-2011 |
| | | TW 201004918 A | 01-02-2010 |
| | | US 2011046283 A1 | 24-02-2011 |
| | | WO 2009146991 A2 | 10-12-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102006026624
- DE 1099523
- DE 3520750
- EP 1256566 A
- WO 2007038489 A
- EP 1063257 B1


### In der Beschreibung aufgeführte Nicht-Patentliteratur


- **GÄCHTER/MÜLLER.** Kunststoffadditive. Carl Hanser-Verlag, 1990, 417-418